# EUROPEAN PATENT APPLICATION

(11) **EP 1 974 727 A1**
(43) Date of publication of application: **01.10.2008**
(21) Application number: 06842989.3
(22) Date of filing: 21.12.2006
(51) Int. Cl.: A61K 31/20, A61P 9/10, A61P 25/28

(54) **THERAPEUTIC AGENT FOR ACUTE CEREBRAL INFARCT**

(30) Priority: 22.12.2005 JP 2005369154
(71) Applicant: ONO PHARMACEUTICAL CO., LTD., Osaka-shi, Osaka 541-8526 (JP)
(72) Inventor: KAJITANI, Hitoshi, Osaka-shi, Osaka 5418526 (JP); FUNAKOSHI, Yosuke, Osaka-shi, Osaka 5418526 (JP); KITAO, Dai, Osaka-shi, Osaka 5418526 (JP)
(74) Representative: Henkel, Feiler & Hänzel
(86) International application number: PCT/JP2006/325481
(87) International publication number: WO 2007/072902

(57) **Abstract**

An agent for treating acute cerebral infarction, comprising (2R)-2-propyloctanoic acid or a salt thereof, which is used for administration after a lapse of time of from about 5 hours to about 72 hours from the onset of the disease is useful for treating acute cerebral infarction, since it safely improves acute cerebral infarction or accompanying symptoms in the patient of acute cerebral infarction, specifically in the patient of acute cerebral infarction defined as a score of 7 to 22 on NIH Stroke Scale.

## Description

### TECHNICAL FIELD

The present invention relates to a method of administering an effective amount of (2R)-2-propyloctanoic acid or a salt thereof for treatment of acute cerebral infarction, after a lapse of time of from about 5 hours to about 72 hours from the onset of the disease, and an agent for use therein.

More specifically, the present invention relates to the method of administering an effective amount of (2R)-2-propyloctanoic acid or a salt thereof for improving neurological symptom, impairment of activities of daily living and/or functional disorder, especially for improving disturbed consciousness, lalopathy and/or impaired limb motor function, accompanying acute cerebral infarction, and the agent for use therein.

### BACKGROUND ART

Examples of diseases which are cause of stroke include cerebral infarction together with cerebral hemorrhage and subarachnoid hemorrhage. Cerebral infarction is a neurodegenerative disease in which anterior blood flow stops by arteriosclerosis of a brain blood vessel or obstruction of a brain blood vessel caused by thrombi to cease feeding of nutrients into brain cells and finally causes the death of nerve cells. Additionally, even when a patient with cerebral infarction has escaped from sudden death, it sometimes leaves serious sequelae such as hemiplegia and aphasia caused by functional disorder of nerve cells.

At present, the thrombolytic therapy which uses tissue-type plasminogen activator (t-PA) or a recombinant thereof (rt-PA), which is the main stream of cerebral infarction treatment, is a markedly useful method for attempting reopening of blood flow by lysing thrombi which are obstructing cerebral blood vessel. However, from the results of a lot of clinical trials, a severe restriction is provided for such a thrombolytic therapy that the agent must be administered within 3 hours after the onset of cerebral infarction, so-called "super-acute period".

Thus, in the treatment of cerebral infarction, there is certain time after the onset of cerebral infarction during which a certain treatment can alleviate degree of cerebral damage, accelerate restoration of functions and improve a long-term result. The acceptable period defined by Pulsinelli et al., as "therapeutic time window" (Ann. NY Acad. Sci. USA, 835, 187-193, 1997) varies depending on the developing condition of collateral circulation and individual case of the disease, and that of the thrombolytic therapy is considered to be roughly about 3 hours after the onset of cerebral infarction.

On the other hand, since pentanoic acid derivatives including (2R)-2-propyloctanoic acid have activity to improve functions of astrocyte, it is known that they are effective for treating stroke and other various diseases. Additionally, it is described that they are orally administered once to several times a day within a range of from 1 to 1,000 mg per one dose per adult, or parenterally administered once to several times a day within a range of from 0.1 to 100 mg per one dose (*e.g*., see the Patent Document 1).

Moreover, it is reported that (2R)-2-propyloctanoic acid is administered to the patient in order to treat cerebral infarction within 2 weeks after the onset of the disease, preferably within from 2 days to within 5 days after the onset of disease, more preferably within 24 hours after the onset of disease, further preferably within 6 hours after the onset of disease (*e.g*., see the Patent Document 2).
[Patent Document 1] Specification of European Patent 0632008
[Patent Document 2] International Publication Pamphlet WO2005/032537

### DISCLOSURE OF THE INVENTION

### PROBLEMS TO BE SOLVED BY THE INVENTION

In general, a pharmaceutical preparation can prevent worsening of the condition of disease and achieve an excellent therapeutic effect when its administration is started at a stage of relatively mild symptom just after the onset of the disease. This is significant particularly in the case of cerebral infarction, and since an irreversibly changed cerebral tissue area periodically expands after the onset of the disease, extremely rapid treatment is necessary. For example, the "International Guidelines for cardiopulmonary resuscitation and emergency cardiovascular care" describes on the t-PA therapy at the time of the onset of cerebral infarction that "one must make an effort to start the t-PA intravenous therapy within 1 hour by finishing clinical evaluation within 10 minutes after arrival of the patient; completing CT scanning within 45 minutes; and further judging adaptation of the treatment". However, it is extremely difficult to carry out all steps of bringing into hospital a patient who fainted away due to cerebral infarction; obtaining a diagnosis as cerebral infarction; and starting an appropriate treatment, within 3 hours. Under such a situation, great concern has been directed toward the development of an agent by which cerebral infarction can be effectively treated even after a lapse of time of 3 hours or more.

### MEANS FOR SOLVING THE PROBLEMS

With the aim of finding out an agent by which cerebral infarction can be effectively treated even after a lapse of time of 3 hours or more from the onset of the disease, the inventors of the present invention have carried out intensive studies and found as a result that (2R)-2-propyloctanoic acid is a compound which fundamentally overturns the theory in the conventional cerebral infarction treatment that its higher cerebral infarction treating effect can be obtained when it is administered after a lapse of time of about 5 hours or more from the onset of the disease, in comparison with the case of administering just after the onset of the disease. The inventors have accomplished the present invention by carrying out the studies further in detail based on this knowledge.

Thus, the present invention relates to :
[1] an agent for treating acute cerebral infarction, comprising (2R)-2-propyloctanoic acid or a salt thereof, which is used for administration after a lapse of time of from about 5 hours to about 72 hours from the onset of the disease;
[2] the agent according to above [1], wherein the treatment of acute cerebral infarction is improvement of neurological symptom, impairment of activities of daily living and/or functional disorder, accompanying acute cerebral infarction;
[3] the agent according to above [1], wherein the treatment of acute cerebral infarction is improvement of disturbed consciousness, lalopathy and/or impaired limb motor function, accompanying acute cerebral infarction;
[4] the agent according to above [1], wherein the acute cerebral infarction is mild or moderate;
[5] the agent according to above [4], wherein the acute cerebral infarction is moderate;
[6] the agent according to above [1], wherein the acute cerebral infarction is defined as a score of 22 or less on NTH Stroke Scale;
[7] the agent according to above [6], wherein the acute cerebral infarction is defined as a score of 7 to 22 on NIH Stroke Scale;
[8] the agent according to above [7], wherein the acute cerebral infarction is defined as a score of 7 to 18 on NIH Stroke Scale;
[9] the agent according to above [1], which is for intravenous administration;
[10] the agent according to above [9], wherein the amount per dose of (2R)-2-propyloctanoic acid or a salt thereof for intravenous administration is within a range of from about 100 mg to about 2000 mg in terms of (2R)-2-propyloctanoic acid;
[11] the agent according to above [10], which is used for continuous administration;
[12] the agent according to above [11], which is used for infusion bag administration;
[13] the agent according to above [11], wherein the continuous administration is carried out for 30 minutes to 90 minutes;
[14] the agent according to above [9], wherein the dose for intravenous administration per 1 kg of body weight of a patient is within a range of from about 2 mg to about 20 mg;
[15] the agent according to above [14], wherein the dose for intravenous administration per 1 kg of body weight of a patient is about 4 mg, about 6 mg, about 8 mg, about 10 mg, about 12 mg, about 15 mg or about 18 mg;
[16] the agent according to above [15], wherein the dose for intravenous administration per 1 kg of body weight of a patient is about 8 mg or about 10 mg;
[17] the agent according to above [1], wherein the administration period is from 1 day to 100 days from the administration after a lapse of time of from about 5 hours to about 72 hours from the onset of the disease;
[18] the agent according to above [17], wherein the administration period is from 1 day to 10 days from the administration after a lapse of time of from about 5 hours to about 72 hours from the onset of the disease;
[19] the agent according to above [18], wherein the administration period is from 5 days to 7 days from the administration after a lapse of time of from about 5 hours to about 72 hours from the onset of the disease;
[20] the agent according to above [1], wherein the administration is carried out once a day;
[21] an agent for treating an acute cerebral infarction defined as a score of 22 or less on NIH Stroke Scale, comprising (2R)-2-propyloctanoic acid,
   which is used for continuous administration for 30 minutes to 90 minutes once a day using infusion bag in an amount per dose of from about 2 mg to about 20 mg per 1 kg of body weight of a patient for intravenous administration,
   in which the administration period is from 1 day to 10 days from the administration after a lapse of time of from about 5 hours to about 72 hours from the onset of the disease;
[22] an agent for improving neurological symptom, impairment of activities of daily living and/or functional disorder, accompanying acute cerebral infarction defined as a score of 7 to 22 on NIH Stroke Scale, comprising (2R)-2-propyloctanoic acid,
   which is used for continuous administration for 30 minutes to 90 minutes once a day using infusion bag in an amount per dose of from about 8 mg or about 10 mg per 1 kg of body weight of a patient for intravenous administration,
   in which the administration period is from 5 day to 7 days from the administration after a lapse of time of from about 5 hours to about 72 hours from the onset of the disease;
[23] an agent for improving disturbed consciousness, lalopathy and/or impaired limb motor function, accompanying acute cerebral infarction defined as a score of 11 to 18 on NIH Stroke Scale, comprising (2R)-2-propyloctanoic acid,
   which is used for continuous administration for 30 minutes to 90 minutes once a day using infusion bag in an amount per dose of about 8 mg or about 10 mg per 1 kg of body weight of a patient for intravenous administration,
   in which the administration period is from 5 day to 7 days from the administration after a lapse of time of from about 5 hours to about 72 hours from the onset of the disease;
[24] a method for treating acute cerebral infarction, which comprises administration of an effective amount of (2R)-2-propyloctanoic acid or a salt thereof to a mammal after a lapse of time of from about 5 hours to about 72 hours from the onset of the disease;
[25] use of (2R)-2-propyloctanoic acid or a salt thereof for the manufacture of an agent for treating acute cerebral infarction which is used for administration after a lapse of time of from about 5 hours to about 72 hours from the onset of the disease; and so forth.

### EFFECT OF THE INVENTION

The method of the present invention and the agent for use therein are safe and possible to significantly improve acute cerebral infarction or accompanying symptoms. The agent of the present invention is useful as medicament, because it is effective in a patient who has been elapsed 3 hours or more after the onset of the disease, which is difficult to treat by the medicaments hitherto known.

### BEST MODE FOR CARRYING OUT THE INVENTION

In the present invention, (2R)-2-propyloctanoic acid is a compound represented by the formula (I): 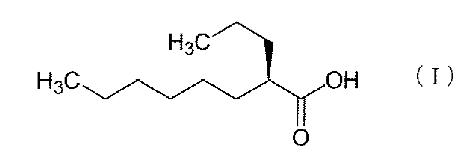 wherein 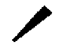 represents β-configuration.

In the present invention, a preferable salt of (2R)-2-propyloctanoic acid is a pharmaceutically acceptable salt. Preferable pharmaceutically acceptable salt is a less-toxic water-soluble salt. Examples of a suitable salt of (2R)-2-propyloctanoic acid include salts with inorganic bases, salts with organic bases, salts with basic natural amino acids and so forth. Preferable examples of the salts with inorganic bases include an alkali metal salt (*e*.*g*., a sodium salt, a potassium salt, a lithium salt, *etc.*), an ammonium salt (*e*.*g*., a tetramethylammonium salt, a tetrabutylammonium salt, *etc.*) and the like. Preferable examples of the salts with organic bases include a salt with an alkylamine (*e*.*g*., methylamine, dimethylamine, trimethylamine, triethylamine, *etc*.), a heterocyclic amine (*e.g*., pyridine, picoline, piperidine, *etc.*), an alkanolamine (*e.g*., ethanolamine, diethanolamine, triethanoalmine, *etc.*), dicyclohexylamine, N,N'-dibenzylethylenediamine, cyclopentylamine, benzylamine, dibenzylamine, phenethylamine, tris(hydroxymethyl)methylamine, N-methyl-D-glucamine and the like. The salt with a basic natural amino acid is not particularly limited, so long as it is a salt with a basic amino acid which exists in nature and can be purified. It is preferable to use, for example, a salt with arginine, lysine, ornithine, histidine or the like.

(2R)-2-propyloctanoic acid or a salt thereof can be prepared by methods known *per se*, for example, the methods described in the specification of EP patent No.0632008, WO 99/58513, WO 00/48982, the specification of JP patent No.3032447, the specification of JP patent No.3084345, WO 2003/051852, WO 2003/097851, WO 2004/092113, WO 2004/110972, WO 2005/105722, methods similar thereto, or the method described in Comprehensive Organic Transformations: A Guide to Functional Group Preparations, 2nd Edition (Richard C. Larock, John Wiley & Sons Inc., 1999) or by appropriate combinations of such methods. The reaction product may be purified by conventional purification methods, for example, by distillation under atmospheric or reduced pressure, high performance liquid chromatography, thin layer chromatography or column chromatography using silica gel or magnesium silicate, or by methods such as washing and recrystallization. Also, if necessary, the product may be subjected to additional treatments such as freeze-drying.

(2R)-2-propyloctanoic acid or a salt thereof to be used in the present invention is not limited to those which are substantially pure single substances, and they may contain impurities (*e*.*g*., byproducts, solvents, starting materials, *etc.* originating form the preparation process, or decomposition products, *etc.*) within the scope acceptable as a drug substance. The content of impurities acceptable as the drug substance varies depending on whether the (2R)-2-propyloctanoic acid is used or a salt thereof is used, and also varies depending on the contained impurities. In the case of (2R)-2-propyloctanoic acid, for example, it is desirable that heavy metals (*e.g*., lead, bismuth, cuprum, cadmium, antimony, tin, mercury, *etc.*) are about 20 ppm or less, the S-form as its optical isomer is about 1.49 % by mass or less, 2-propanol and heptane as the residual solvents are about 5000 ppm or less in total, and the moisture is about 0.2 % by mass or less. Particularly, (2R)-2-propyloctanoic acid having an optical purity of about 99 % e.e. or more, more particularly, (2R)-2-propyloctanoic acid having an optical purity of about 99.3 % e.e. or more, is suitable as the (2R)-2-propyloctanoic acid to be used in the present invention.

The present invention discloses the method comprising administering an effective amount of above-described (2R)-2-propyloctanoic acid or a salt thereof (preferably (2R)-2-propyloctanoic acid) after a lapse of time of from about 5 hours to about 72 hours from the onset of the disease, preferably in the amount per dose of (2R)-2-propyloctanoic acid or a salt thereof within a range of from about 100 mg to about 2000 mg, in order to treat acute cerebral infarction, more specifically to improve neurological symptom, impairment of activities of daily living and/or functional disorder, accompanying acute cerebral infarction, in particular to improve disturbed consciousness, lalopathy and/or impaired limb motor function, accompanying acute cerebral infarction (hereinafter sometimes abbreviated as the method of the present invention) and the agent for treating acute cerebral infarction which is for use therein (the agent for treating acute cerebral infarction comprising (2R)-2-propyloctanoic acid or a salt thereof which is used for administration after a lapse of time of from about 5 hours to about 72 hours from the onset of the disease which is hereinafter sometimes abbreviated as the agent of the present invention).

In the present invention, (2R)-2-propyloctanoic acid or a salt thereof is administered after a lapse of time of from about 5 hours to about 72 hours from the onset of acute cerebral infarction to an intended mammal (*e.g*., human or non-human animal (*e.g*., monkey, sheep, cattle, horse, dog, cat, rabbit, rat, mouse, *etc*.), preferably human (patient)). Additionally, the "administration" used herein means the first administration of (2R)-2-propyloctanoic acid or a salt thereof after the onset of the disease, what is called "first dose". The time of first dose is not limited, so long as it is after a lapse of time of from about 5 hours to about 72 hours from the onset of the disease. For example, the preferable time of first dose is after a lapse of time of from about 5 hours to about 48 hours from the onset of the disease, more preferable time of first dose is after a lapse of time of from about 5 hours to about 24 hours from the onset of the disease, and further preferable time of the first dose is after a lapse of time of from about 5 hours to about 12 hours from the onset of the disease. Above all, the time after a lapse of time of from about 5.5 hours to about 6 hours from the onset of the disease is preferable, as shown in Examples described later.

In this connection, the "onset of the disease" in the present invention means development of a symptom to such a degree that it can be diagnosed as "onset of the disease" from the medical point of view, or that point of time. Although the term "onset of the disease" having a temporal means may be any point of time so long as it is a point of time when symptoms are diagnosed as the "onset of the disease" from the medical point of view, it is used in general as a meaning of a point of time when the first infarction symptom was found, or a point of time when the infarction symptom started, as shown in the <Inclusion Criteria> (c) described in Example 1 which is described later for example.

In the present invention, the method of administering (2R)-2-propyloctanoic acid or a salt thereof is not limited, so long as it is the method which enables to administer (2R)-2-propyloctanoic acid or a thereof to intended animal (preferably patient) in order to treat acute cerebral infarction. However, in order to obtain preferable effect of treatment against acute cerebral infarction, it is preferable that the administration is carried out in accordance with, for example, administration period, administration frequency, route of administration, dose, *etc.* as described below.

Regarding the administration period, it may be administered continuously for the optional number of days starting from the point of the administration carried out after about 5 hours to about 72 hours from the onset of the disease (the aforementioned "first dose"). Additionally, it may be administered intermittently by setting appropriate drug cessation periods as needed. Examples of the illustrative administration period include a period of from I day to 100 days. Preferable administration period is, for example, a period of from 1 day to 14 days or a period of from 1 day to 10 days. More preferable administration period is, for example, 3 days, 4 days, 5 days, 6 days or 7 days, and most preferable administration period is, for example, 5 days, 6 days or 7 days. Particularly, as is shown in the Examples which are shown later, an administration period of 5 days or 7 days is preferable.

Regarding the administration frequency, it may be the optional number of times during the aforementioned administration period. Additionally, the frequency may be changed depending on the condition of the intended mammal (preferably a patient) and other reasons. Examples of the illustrative administration frequency per day include from 1 to 5 times. Preferable administration frequency per day is, for example, from 1 to 3 times. More preferred administration frequency per day is, for example, 1 or 2 times. Most preferable administration frequency per day is, for example, once a day.

Although the route of administration is not particularly limited so long as the agent can be administered to the intended mammal (preferably a patient), a route of administration which enables possible quick transfer of the agent into the brain, such as intravenous administration or the like, is desirable. For the intravenous administration, it is preferable to use the agent by preparing it into dosage forms which can be intravenously administered such as injections and infusions. By making into a dosage form which can be administered into a vein, it becomes possible to quickly obtain the acute cerebral infarction-treating effect of (2R)-2-propyloctanoic acid or a salt thereof. Additionally, when the (2R)-2-propyloctanoic acid or a salt thereof prepared as such a dosage form which can be administered into a vein is continuously administered into a vein as a continuous administration preparation, for example using a syringe, infusion bag or the like, avoidance of side effects associated with rapid increase of blood concentration and, as needed, control of blood concentration and the like are possible. Although the period of time for continuous administration is not particularly limited and may be changed depending on the condition of the patient and other reasons, it is preferable to carry out the continuous administration for example from about 0.5 hour (about 30 minutes) to about 4 hours (about 240 minutes), preferably from about 0.5 hour (about 30 minutes) to about 1.5 hours (about 90 minutes), particularly preferably about 1 hour (about 60 minutes).

Although the dosage may be any amount so long as it is an amount by which (2R)-2-propyloctanoic acid or a salt thereof shows its efficacy without showing significant toxicity in the living body, as described in the foregoing, for example, from about 100 mg to about 2,000 mg is preferable. The agent can be used without limitation within such a range. Particularly, when intravenous administration is carried out as injections or infusions as described in the above, it is preferable to determine the dosage based on the body weight of the intended mammal (preferably a patient). For example, when (2R)-2-propyloctanoic acid is intravenously administered, it is preferbale to administer for example from about 2 mg to about 20 mg of the same per 1 kg of body weight of the intended mammal (preferably a patient). Examples of illustrative dosage include about 4 mg, about 6 mg, about 8 mg, about 10 mg, about 12 mg, about 15 mg or about 18 mg, per 1 kg of body weight. Examples of more preferable dose include about 4 mg, about 6 mg, about 8 mg, about 10 mg or about 12 mg, per 1 kg of body weight. Examples of most preferable dose include about 8 mg or about 10 mg, per 1 kg of body weight. Additionally, when a salt of (2R)-2-propyloctanoic acid is intravenously administered, the dosage shown in the above is desirable as the amount of (2R)-2-propyloctanoic acid.

Examples of the method for administering an effective amount of (2R)-2-propyloctanoic acid or a salt thereof for the treatment of acute cerebral infarction according to the present invention includes as a preferable example a method in which from about 2 mg to about 20 mg of (2R)-2-propyloctanoic acid or a salt thereof is continuously administered, as the amount of (2R)-2-propyloctanoic acid per 1 kg of body weight of a patient, into a vein using an infusion bag at a frequency of from 1 to 3 times a day (preferably once a day) for a period of from about 0.5 hour to about 1.5 hours (preferably about 1 hour), during an administration period of from 1 to 10 days starting from the administration after a lapse of time of from about 5 hours to about 72 hours from the onset of the disease, or the like. More illustratively, as is described in the Examples which are described later, a method in which about 8 mg or about 10 mg of (2R)-2-propyloctanoic acid or a salt thereof, as the amount of (2R)-2-propyloctanoic acid per 1 kg of body weight of a patient, is continuously administered into a vein using an infusion bag once a day for about 1 hour, during an administration period of from 5 to 7 days starting from the administration after a lapse of time of from about 5 hours to about 72 hours from the onset of the disease is preferable.

In the present invention, although the subject to which (2R)-2-propyloctanoic acid or a salt thereof is administered is not particularly limited, so long as it is a mammal (*e.g*., human or a non-human animal (*e.g*., monkey, sheep, cattle, horse, dog, cat, rabbit, rat, mouse or the like)) which developed the acute cerebral infarction, it is particularly preferable to select human as the object. Although the person (patient) who developed acute cerebral infarction may be any patient, a patient of from mild to moderate, particularly moderate, acute cerebral infarction is preferable. Classification of such cases can be carried out using various diagnostic tests conventionally known in said technical field for judging the degree of damage in cerebral infarction, such as Glasgow Outcome Scale (GOS), Glasgow Coma Scale (GCS), Rankin Scale (RS), modified Rankin Scale (mRS), Disability Rating Scale (DRS), Hemispheric Stroke Scale, Scandinavian Stroke Scale, Canadian Stroke Scale, Japan Stroke Scale (JSS), Japan Coma Scale (JCS), Stroke Impact Scale (SIS), NIH Stroke Scale (NIHSS) and the like. These diagnostic tests can be carried out using conventionally known methods. For example, illustrative scoring of the NIH Stroke Scale is carried out based on the scoring table (Question #1a, Question #1b, Question #1c and Question #2 to Question #11) described in the following.

### <NIH Stroke Scale scoring table>

### Question #1a : level of consciousness

The investigator must choose a response if a full evaluation is prevented by such obstacles as an endotracheal tube, language barrier, orotracheal trauma/bandages. Score 3 is given only if the patient makes no movement (other than reflexive posturing) in response to noxious stimulation.
score 0 : Alert. keenly responsive.
score 1 : Not alert. But arousable by minor stimulation to obey, answer, or respond.
score 2 : Requires repeated stimulation to attend, or is obtunded and requires strong or painful stimulation to make movements (not stereotyped).
score 3 : Responds only with reflex motor or autonomic effects or totally unresponsive, flaccid, and areflexic.

### Question #1b : level of consciousness (Questions)

The patient is asked the month and his/her age. The answer must be correct - there is no partial credit for being close. Aphasic and stuporous patients who do not comprehend the questions will score 2. Patients unable to speak because of endotracheal intubation, orotracheal trauma, severe dysarthria from any cause, language barrier, or any other problem not secondary to aphasia are given score 1. It is important that only the initial answer be graded and that the examiner not "help" the patient with verbal or non-verbal cues.
score 0 : Answers both questions correctly.
score 1 : Answers one question correctly.
score 2 : Answers neither question correctly.

### Question #1c : level of consciousness (Commands)

The patient is asked to open and close the eyes and then to grip and release the hand. Substitute another one step command if the hands cannot be used. Credit is given if an unequivocal attempt is made but not completed due to weakness. If the patient does not respond to command, the task should be demonstrated to him or her (pantomime). Patients with trauma, amputation, or other physical impediments should be given suitable one-step commands. Only the first attempt is scored.
score 0 : Performs both tasks correctly.
score 1 : Performs one task correctly.
score 2 : Performs neither task correctly.

### Question #2 : Best Gaze

Only horizontal eye movements will be tested. Voluntary or reflexive (oculocephalic) eye movements will be scored, but caloric testing is not done. If the patient has a conjugate deviation of the eyes that can be overcome by voluntary or reflexive activity, the score will be score 1. If a patient has an isolated peripheral nerve paresis (III, IV or VI), score 1. Gaze is testable in all aphasic patients. Patients with ocular trauma, bandages, pre-existing blindness, or other disorder of visual acuity or fields should be tested with reflexive movements, and a choice made by the investigator. Establishing eye contact and then moving about the patient from side to side will occasionally clarify the presence of a partial gaze palsy.
score 0 : Normal.
score 1 : Gaze is abnormal in one or both eyes, but forced deviation or total gaze paresis is not present.
score 2 : "Doll's eye" Forced deviation, or total gaze paresis not overcome by the oculocephalic maneuver.

### Question #3 : Visual

Visual fields (upper and lower quadrants) are tested by confrontation, using finger counting or visual threat, as appropriate. Patients may be encouraged, but if they look at the side of the moving fingers appropriately, this can be scored as normal. If there is unilateral blindness or enucleation, visual fields in the remaining eye are scored. Score 1 only if a clear-cut asymmetry, including quadrantanopia, is found. If patient is blind from any cause, score 3.
score 0 : No visual loss.
score 1 : Partial hemianopia.
score 2 : Complete hemianopia.
score 3 : Bilateral hemianopia (blind including cortical blindness).

### Question #4 : Facial Palsy

Ask or use pantomime to encourage the patient to show teeth or raise eyebrows and close eyes. Score symmetry of grimace in response to noxious stimuli in the poorly responsive or non-comprehending patient. If facial trauma/bandages, orotracheal tube, tape or other physical barriers obscure the face, these should be removed to the extent possible.
score 0 : Normal symmetrical movements.
score 1 : Flattened nasolabial fold, asymmetry on smiling.
score 2 : Total or near-total paralysis of lower face.
score 3 : Absence of facial movement in the half of face.

### Question #5 : Motor Arm

Upper limb is extended at the 90 degrees (if sitting) or the 45 degrees (if supine). The aphasic patient is encouraged using urgency in the voice and pantomime, but not noxious stimulation. Each limb is tested in turn, beginning with the non-paretic arm. Only in the case of amputation or joint fusion at the shoulder, the examiner should record the score 9, and clearly write the explanation for this choice.
score 0 : Limb holds 90 (or 45) degrees for full 10 seconds.
score 1 : Limb holds 90 (or 45) degrees, but drifts down before full 10 seconds; does not hit bed.
score 2 : Some effort against gravity; limb cannot get to or maintain 90 (or 45) degrees.
score 3 : No effort against gravity; limb falls to bed.
score 4 : No movement.
score 9 : Amputation or joint fusion.

### Question #6 : Motor Leg

Lower limb is placed at the 30 degrees (always tested supine). The aphasic patient is encouraged using urgency in the voice and pantomime, but not noxious stimulation. Each limb is tested in turn, beginning with the non-paretic leg. Only in the case of amputation or joint fusion at the hip, the examiner should record the score 9, and clearly write the explanation for this choice.
score 0 : Leg holds 30 degrees for full 5 seconds.
score 1 : Leg holds 30 degrees, but drifts down before full 5 seconds; does not hit bed.
score 2 : Some effort against gravity; but leg falls.
score 3 : No effort against gravity; leg falls to bed immediately.
score 4 : No movement.
score 9 : Amputation or joint fusion.

### Question #7 : Limb ataxia

The finger-nose-finger and heel-shin tests are performed on both sides. Test with eyes open. In case of visual defect, ensure testing is done in intact visual field. Ataxia is scored only if present out of proportion to weakness. Score 0 is recorded in the patient, who cannot understand or is paralyzed. Only in the case of amputation or joint fusion, the examiner should record the score 9, and clearly write the explanation for this choice. In case of blindness, test by having the patient touch nose from extended arm position.
score 0 : Absent.
score 1 : Present in one limb.
score 2 : Present in two limbs.
score 9 : Amputation or joint fusion.

### Question #8 : Sensory

Evaluate by sensation or grimace to pinprick when tested, or withdrawal from noxious stimulus in the disturbed consciousness or aphasic patient. The examiner should test as many body areas (arms, legs, trunk, face) as needed to accurately check for hemisensory loss. A score 2, "severe or total sensory loss", should only be given when a severe or total loss of sensation can be clearly demonstrated. Stuporous and aphasic patients will, therefore, probably score 1 or 0. The patient with brainstem stroke who has bilateral loss of sensation is scored 2. If the patient does not respond or is quadriplegic, score 2 is given. Patients in a coma are automatically given score 2 on this item.
score 0 : Normal.
score 1 : Patient feels pinprick is less sharp; or there is a loss of superficial pain with pinprick, but patient is aware of being touched.
score 2 : Patient is not aware of being touched.

### Question #9 : Best Language

A great deal of information about comprehension will be obtained during the preceding sections of the examination. For this scale item, the patient is asked to describe what is happening in the attached picture, to name the items on the attached naming sheet and to read from the attached list of sentences. Comprehension is judged from responses here, as well as to all of the commands in the preceding general neurological exam. If visual loss interferes with the tests, ask the patient to identify objects placed in the hand, repeat, and produce speech. The intubated patient should be asked to write. The examiner must choose a score for the patient with stupor or limited cooperation, but a score 3 should be used only if the patient is mute and follows no one-step commands.
score 0 : Normal.
score 1 : Some obvious loss of fluency or facility of comprehension, without significant limitation on ideas expressed or form of expression. Reduction of speech and/or comprehension, however, makes conversation about provided materials difficult or impossible. Examiner can identify the answer from patient's response.
score 2 : All communication is through fragmentary expression; great need for inference, questioning, and guessing by the listener. Range of information that can be exchanged is limited; listener carries burden of communication. Examiner cannot identify the answer from patient's response.
score 3 : No usable speech or auditory comprehension.

### Question #10 : Dysarthria

If patient is thought to be normal, an adequate sample of speech must be obtained by asking patient to read or repeat words from the attached list. If the patient is aphasia, the clarity of articulation of spontaneous speech can be rated. Only if the patient is intubated or has other physical barriers to producing speech, the examiner should record the score 9, and clearly write an explanation for this choice. Do not tell the patient why he or she is being tested.
score 0 : Normal.
score 1 : Patient slurs at least some words and, at worst, can be understood with some difficulty.
score 2 : Patient's speech is so slurred as to be unintelligible for the examiner.
score 9 : Intubated or other physical barrier.

### Question #11 : Extinction and Inattention

Sufficient information to identify neglect may be obtained during the prior testing. If the patient has a severe visual loss preventing visual double simultaneous stimulation, and the cutaneous stimuli are normal, the score is normal. If the patient has aphasia but does appear to attend to both sides, the score is normal. The presence of visual spatial neglect or anosagnosia may also be taken as evidence of abnormality. Since the abnormality is scored only if present, the item is never untestable.
score 0 : No abnormality.
score 1 : Visual, tactile, auditory, spatial, or personal inattention. Extinction to bilateral simultaneous stimulation in one of the sensory modalities.
score 2 : Profound hemi-inattention or extinction to more than one modality. Does not recognize own hand or orients to only one side of space.

Additionally, for example, illustrative scoring of the Japan Coma Scale is carried out based on the following evaluation method.

### <Japan Coma Scale evaluation method>

0 : Absolutely normal.
   I. Awake without any stimuli
1 : Almost clear consciousness, but not completely clear.
2 : Does not understand time, person and place (disorientation).
3 : Does not understand his or her name and the date of birth.
   II. Rousable using stimuli
10 : Easily opens eyes by usual calling.
20 : Opens eyes by a large voice or shaking of the body.
30 : Barely opens eyes when calling is repeated with applying pain or stimulation.
   III. Unrousable using any forceful stimuli
100 : Makes an action against pain stimulation as if brushing aside.
200 : Slightly moves hand and foot or makes a wry face against pain stimulation.
300 : Does not react at all with pain stimulation.

Further in addition, for example, illustrative evaluation of the Glasgow Coma Scale is carried out on the basis of a total of 15 scores, based on the following evaluation method.

### <Glasgow Coma Scale evaluation method>

1. Eye Opening Response
   4 points : Opens spontaneously.
   3 points : Opens to verbal command.
   2 points : Opens to pain.
   1 point: None
2. Verbal Response
   5 points: Oriented.
   4 points : Confused conversation.
   3 points : Only disordered words.
   2 points: Incomprehensible speech.
   1 point: None
3. Motor Response
   6 points: Obeys commands for movement.
   5 points: Purposeful movement.
   4 points: Withdraws from pain.
   3 points: Abnormal flexion.
   2 points : Extensor response.
   1 point: None

In the present invention, the "acute cerebral infarction of mild or moderate" means those which show roughly 22 or less of scores on the aforementioned NIH Stroke Scale. Additionally, the "acute cerebral infarction of mild" means those which show roughly 6 or less of scores on the aforementioned NIH Stroke Scale, and the "acute cerebral infarction of moderate" means those which show roughly from 7 to 22 of scores on the aforementioned NIH Stroke Scale.

In the present invention, the (2R)-2-propyloctanoic acid or a salt thereof can show further superior therapeutic effect on the "acute cerebral infarction of mild or moderate", particularly on the "acute cerebral infarction of moderate". As described in the foregoing, since the "acute cerebral infarction of moderate" means those which show from 7 to 22 of scores on the NIH Stroke Scale, the (2R)-2-propyloctanoic acid or a salt thereof can exert superior therapeutic effect upon any acute cerebral infarction which show a score within this range. For example, it can show superior therapeutic effect upon cerebral infarction which shows a score within an optional range such as from 7 to 18, from 11 to 22 or from 11 to 18 on the NIH Stroke Scale. The (2R)-2-propyloctanoic acid or a salt thereof can show further superior therapeutic effect on an acute cerebral infarction of relatively slight degree which show for example from 7 to 18 of scores, among acute cerebral infarctions which show from 7 to 22 of scores on the NIH Stroke Scale.

Additionally, when an index other that the NIH Stroke Scale is used, a cerebral infarction which is evaluated as from 0 to II-30 on the aforementioned Japan Coma Scale can also be regarded as the "acute cerebral infarction of mild or moderate". The (2R)-2-propyloctanoic acid or a salt thereof can show superior therapeutic effect also on a patient evaluated as from 0 to II-30 on the aforementioned Japan Coma Scale, particularly on a patient evaluated as from 0 to I-3 or from I-1 to II-30.

In the present invention, in the case of human as the object to which (2R)-2-propyloctanoic acid or a salt thereof is administered, it is desirable that age of the patient of acute cerebral infarction is less than 80. When age of the patient is less than 80, preferably less than 75, the (2R)-2-propyloctanoic acid or a salt thereof can show further superior therapeutic effect.

In the present invention, whether or not the (2R)-2-propyloctanoic acid or a salt thereof is effective for the treatment of acute cerebral infarction may be evaluated, using various diagnostic tests conventionally known in said technical field similar to the case described in the foregoing, or evaluated using a testing method which detects a physical abnormality in the brain, such as CAT scanning, MRI (magnetic resonance imaging), measurement of intracranial pressure or the like. Alternatively, it can be carried out by optionally combining these. In general, in the clinical trial which is carried out using a patient of cerebral infarction, evaluation of the efficacy is carried out using the aforementioned diagnostic tests as the main evaluation items. Additionally, as needed, for example, level of consciousness, motor function, Barthel Index, general safety, outcome, head CT findings, head MRI findings, blood pressure, pulse, body temperature, general clinical laboratory tests and the like conventionally known evaluation items may be evaluated as secondary evaluation items and used for the evaluation of efficacy, alone or in combination with the main evaluation items.

In the present invention, (2R)-2-propyloctanoic acid or a salt thereof shows excellent therapeutic effect also on symptoms accompanied by acute cerebral infarction such as neurological symptom, impairment of activities of daily living, functional disorder, and the like. These symptoms include, for example, an impaired limb motor function (*e.g*., contlateral hemiplegia, contlateral quadriplegia, alternating hemiplegia, bilateral quadriplegia, incomplete hemiplegia, numbness of upper and lower limbs, powerlessness, motor paralysis, locked-in syndrome or the like), a sensory disturbance (*e.g*., hypesthesia, facial paralysis or the like), a lalopathy (*e.g*., aphasia, dysarthria, tongue paralysis, tongue deviation, agnosia, apraxia, alexia, akinetic mutism, severe disturbance of memory or the like), a visual impairment (*e.g.*, a transient blindness (*e.g*., amaurosis fugax or the like), nystagmus, double vision, impaired eyesight, homonymous hemianopsia, eye motor paralysis, total blindness or the like), a disorder of balance sense (*e.g*., cerebellar ataxia, vertigo, ear noises, ambulation difficulty or the like), eating disorder and dysphagia (*e.g*., nausea, vomiting or the like), a disturbed consciousness (*e.g*., coma, spasm, mental disorder or the like), dyspnoea and the like. Among these symptoms, the (2R)-2-propyloctanoic acid or a salt thereof shows superior therapeutic effect particularly for a disturbed consciousness, lalopathy and an impaired limb motor function, accompanied by acute cerebral infarction. Its effect on the lalopathy and impaired limb motor function is evident also from the examples which are described later. In this connection, the term "treatment" as used herein include the "treatment" of these disorders.

In the present invention, (2R)-2-propyloctanoic acid or a salt thereof can be used as concomitant drug in combination with other agent for treating cerebral infarction. The other agent like this includes, for example, warfarin, heparin, fasudil hydrochloride hydrate, argatroban, sodium ozagrel, alteplase, alprostadil alfadex, cilostazol, batroxobin, tisokinase, levacecarnine hydrochloride, dilazep dihydrochloride, tacrolimus hydrate, edaravone, monteplase, sarpogrelate hydrochloride, pamiteplase, clopidogrel sulfate, aspirin, anti-GPIIb/IIIa antibody (abciximab), atorvastatin calcium, bifemelane, citicoline, DMP-647, dipyridamole + ASA (combination of drugs), activated blood coagulation VII factor (Factor VIIa), ximelagatran, idebenone, indobufen, monosialoganglioside GM-1, nicotinic acid, nimodipine, eptotermin alfa, bortezomib, perindopril erbumine, propentofylline, rasagiline mesylate, triflusal, aniracetam, ramipril, pravastatin + aspirin (combination of drugs), danaparoid sodium, epoprostenol, minaprine, pioglitazone, nicaraven, acetylsalicylic acid (ASA), efaproxiral sodium, disufenton sodium, prasugrel, ancrod, dexanabinol, desmoteplase, MC-1, idraparinux sodium, CPC-211, 3APS, YY-280, LY-293558, SUN-N4057, V-10153, traxoprodil mesylate, IL-1 receptor antagonist, odiparcil, NOX-100, DP-b99, S-0139, EN-100, microplasmin, Bay-59-7939, SGS-111, INO-1001, PAN-811, AX-200, tissue plasminogen activator (t-PA), TG-100-115, BIO-001, S-18986, SEMAX, KAI-9803, TP-10, BGC-728, BIII-890-CL, neuronal transplant cells, DHEAS, AJW-200, SUN-N8075, TS-011, E-2051, ONO-2231, DY-9760e, MLN-519, SA-4503, SB-234551, AEOL-10150, NMED-160, HF-0220, PGX-100, BMS-554216, GFT-46, enecadin, NH-02D, OFNE, NMDA/glycine antagonists, DF-1681, CI-1034, BAY-38-7271, 813893, TP-920 1, and so forth.

Among these concomitant agents, a combination of a thrombolytic agent including t-PA with (2R)-2-propyloctanoic acid or a salt thereof is particularly preferably used. Since it is preferable to administer (2R)-2-propyloctanoic acid or a salt thereof after a lapse of time of from about 5 hours to about 72 hours from the onset of acute cerebral infarction and it is preferable to administer a thrombolytic agent including t-PA within about 3 hours from the onset of the disease, it can be used also as, for example, "a pharmaceutical preparation which comprises a combination of an acute cerebral infarction treating agent that comprises a thrombolytic agent and is administered within about 3 hours from the onset of the disease and another acute cerebral infarction treating agent which comprises (2R)-2-propyloctanoic acid or a salt thereof and is administered after a lapse of time of from about 5 hours to about 72 hours from the onset of the disease". Namely, it is possible to use a combination of administration methods in which a thrombolytic agent (*e.g*., urokinase, abbokinase (tissue culture urokinase), a natural or recombinant t-PA (*e.g.*, tisokinase, alteplase, monteplase, pamiteplase or the like), a proplasminogen activator (*e.g*., nasaruplase or the like) or the like) or the like) is used at an ultra-acute phase (*e.g.*, within about 3 hours from the onset of the disease or the like), and then (2R)-2-propyloctanoic acid or a salt thereof is administered. When administration is carried out by such a combination, the dose of t-PA and its administration method may be the same as the case of single administration. For example, the dose and administration method of t-PA alone are, for example, from about 0.6 mg to about 0.9 mg (preferably about 0.6 mg or about 0.9 mg) per 1 kg of body weight is administered parenterally, preferably intravenously, within 3 hours from the onset of cerebral infarction. More illustratively, 10% of the total amount to be administered is rapidly administered within few minutes (*e.g*., from about 1 minute to about 2 minutes), and then the rest is administered for about 1 hour. Accordingly, when an agent comprising (2R)-2-propyloctanoic acid or a salt thereof is used in combination with t-PA, for example, t-PA may be administered by the above-mentioned method within 3 hours after onset of cerebral infarction, and then (2R)-2-propyloctanoic acid or a salt thereof may be administered after a lapse of time of from about 5 hours to about 72 hours from the onset of the disease, in accordance with the aforementioned administration period, administration frequency, dose and route of administration of (2R)-2-propyloctanoic acid or a salt thereof

In this connection, according to the present invention, "a method for treating acute cerebral infarction by delayed administration" can be disclosed as a new approach of the therapeutic method of acute cerebral infarction. In such method, a pharmaceutical preparation from which excellent therapeutic effect can be obtained even for a patient who passed 3 hours or more from the onset of cerebral infarction, by carrying out preferably after a lapse of time of from about 5 hours to about 72 hours from the onset of the disease (hereinafter, to be referred to as delayed administration type acute cerebral infarction treating agent) is used. Examples of the agent to be used in the delayed administration type acute cerebral infarction treating agent include the agents developed for the purpose of treating cerebral infarction which are a group of compounds which do not directly act upon the coagulation-fibrinolysis system of blood in addition to the (2R)-2-propyloctanoic acid or a salt thereof disclosed in the present invention. Examples of such compounds include fasudil hydrochloride hydrate, levacecarnine hydrochloride, dilazep dihydrochloride, tacrolimus hydrate, edaravone, atorvastatin calcium, bifemelane, citicoline, idebenone, monosialoganglioside GM-1, nicotinic acid, eptotermin alfa, bortezomib, perindopril erbumine, propentofylline, rasagiline mesylate, aniracetam, ramipril, pravastatin + aspirin (combination of drugs), minaprine, pioglitazone, nicaraven, acetyl salicylate (ASA), efaproxiral sodium, disufenton sodium, dexanabinol, MC-1, CPC-211, 3APS, LY-293558, SUN-N4057, traxoprodil mesylate, IL-1RA(IL-1 receptor antagonist), NOX-100, DP-b99, S-0139, EN-100, SGS-111, INO-1001, PAN-811, AX-200, TG-100-115, BIO-001, S-18986, SEMAX, KAI-9803, TP-10, BIII-890-CL, DHEAS, SUN-N8075, TS-011, E-2051, ONO-2231, DY-9760e, MLN-519, SA-4503, SB-234551, AEOL-10150, NMED-160, HF-0220, PGX-100, BMS-554216, GFT-46, enecadin, NH-02D, OFNE, DF-1681, BAY-38-7271 and the like. Similar to the administration method of the (2R)-2-propyloctanoic acid or a salt thereof disclosed in the present invention, when pharmaceutical preparations comprising these agents are used in a treatment by administering after a lapse of time of 3 hours or more, preferably after a lapse of time of from about 5 hours to about 72 hours, from the onset of cerebral infarction, it is included in the "method for treating acute cerebral infarction by delayed administration".

### [Toxicity]

As is also shown in Examples which are described later, toxicity of (2R)-2-propyloctanoic acid or a salt thereof is markedly low, so that it can be judged that (2R)-2-propyloctanoic acid or a salt thereof is sufficiently safe so long as it is used in a mammal, particularly in human, particularly by the use and dose disclosed in the present invention.

### [Application to Medicament]

The methods of the present invention and the agent of the present invention for use therein can be used in mammals (*e.g*., human and non-human animals such as monkey, sheep, cattle, horse, dog, cat, rabbit, rat, mouse and the like). Particularly, suitable effect for treating acute cerebral infarction can be obtained by intravenously administering it (preferably continuous administration) to a mammal, preferably human, by the dosage regimen disclosed in the present invention. The dosage regimen disclosed in the present invention is suitable for obtaining the effect to improve various symptoms of acute cerebral infarction patients. The dosage regimen disclosed in the present invention is able to overcome the limitation of administration period of time of therapeutic agents conventionally used in the cerebral infarction therapy such as the t-PAand the like, and the effect obtained by said dosage regimen is markedly excellent.

In administering (2R)-2-propyloctanoic acid or a salt thereof to the living body, although any dosage form of a composition may be used as long as it can administer (2R)-2-propyloctanoic acid or a salt thereof into the living body, in general, it is used by preparing into a dosage form which is used in the systemic administration of agents, pharmaceutical preparations for oral administration use such as tablets, capsules and the like or preparations for parenteral administration use such as injections and the like. According to the agent of the present invention, as described in the foregoing, particularly when viewpoints of easy administration to a patient and of immediate action and blood concentration management and the like are taken into consideration, dosage forms which can be administered into a vein, such as injections, injections for continuous administration use (so-called transfusions) and the like are preferable.

Examples of the materials which are used when the (2R)-2-propyloctanoic acid or a salt thereof is prepared into pharmaceutical compositions such as injections or transfusions and the like include metal salts generally used in injections (*e.g*., sodium phosphate, disodium hydrogenphosphate, sodium carbonate, sodium sulfite and the like) and pH adjusting agents (*e.g*., sodium hydroxide and the like), as well as stabilizers, surfactants, buffer agents, solubilizing agents, antioxidants, antifoaming agents, tonicity agents, emulsifying agents, suspending agents, preservatives, soothing agents, solubilizing agents, solubility assisting agents and the like described in "Iyakuhin Tenkabutsu Jiten" (edited by Japan Pharmaceutical Additives Association), published in 2000 by Yakuji Nippo-sha and the like, and also electrolytes (*e.g*., sodium chloride, potassium chloride, calcium chloride, sodium lactate, sodium dihydrogenphosphate, sodium carbonate, magnesium carbonate and the like), saccharides (*e.g*., glucose, fructose, sorbitol, mannitol, dextran and the like), protein amino acids (*e.g*., glycine, aspartic acid, lysine and the like), vitamins (*e.g*., vitamin B₁, vitamin C and the like) and the like components and the like which are generally used in transfusions.

### EXAMPLES

Hereinafter, with reference to Examples and Formulation Examples, although clinical effects of (2R)-2-propyloctanoic acid or a salt thereof on cerebral infarction patients using the dosage regimen (DOSAGE and ADMINISTRATION) of the present invention are described in detail, the present invention is not limited thereto.

### Example 1:

As a clinical trial, a double blind placebo-controlled parallel group study was carried out under the following conditions with patients of acute cerebral infarction as the object.

### [STUDY POPULATION]

A total of 841 patients of acute cerebral infarction who meets the following inclusion criteria and does not meet any of the exclusion criteria.

### <Inclusion Criteria>

(a) A male or female patient of 18 or more years old;
(b) A patient having a diagnosis for an acute cortical ischemic stroke (*e.g*., having at least one of cortical symptoms such as aphasia, behavioral incapacitation, anosognosia, neglect, visuospatial disorder and the like);
(c) A patient who undergoes randomization by the study within 6 hours from the first infarction symptom and undergoes the first administration by the blind test (When the patient awoke under an infarction symptom which was not observed before sleeping, start of the infarction symptom is regarded as the started time of sleeping. When the started time is not clear, start of the infarction symptom is regarded as the time when the patient was lastly normal.);
(d) A patient in which measurable neurological deficit according to the infarct area was found for at least 60 minutes. The deficit should be continued under a state of the absence of a clinically significant change during the period of from the onset of the disease to the first administration in the blind test;
(e) A patient having CT findings or MRI findings equivalent to the case of receiving a diagnosis as acute ischemic stroke. As a condition to be considered, the screening/verification test by the CT or MRI may be carried out after the first administration (however, before the second administration) (when a conclusion cannot be obtained by the first CT, a CT scanning for confirmation should be carried out before the next administration);
(f) A patient defined as a score of from 11 to 22 on NIH Stroke Scale, within 1 hour before the first administration;
(g) A patient defined as a score of 0 (absolutely no symptom) or 1 (although there is a symptom, there is no serious disorder: all daily works can be carried out) on modified Rankin Scale before infarction;
(h) Informed consent was obtained from the patient or his or her (recognized by local regulations) formal representative.

### <Exclusion Criteria>

(a) A patient whose body weight exceeded 125 kg (275 pounds);
(b) A patient in which a non-ischemic mechanism so-called acute neurosis due to a brain edema wherein a clinically significant mass effect was recognized, a midline shift with the compression of ventricle, a subarachnoid hemorrhage, primary intracerebral and/or intraventricular hemorrhage was confirmed by CT scanning and/or MRI findings;
(c) A patient who meets all of the following three:
   - Reduction of the level of consciousness (a score of 2 or more in Question #1a of NIH Stroke Scale);
   - Fixed partial deviation or complete gaze palsy (a score of 2 in Question #2 of NIH Stroke Scale); and
   - Serious hemiplegia of upper limbs and lower limbs (akinesia) (namely, a score of 4 in Question #5 of NIH Stroke Scale concerned in the motion of arms and a score of 4 in Question #6 of NIH Stroke Scale concerned in the motion of legs);
(d) A patient in which a rapidly recovering neurological sign or symptom can be observed by physician-in-charge;
(e) A patient who has a serious complication or terminal systemic disease (HIV/AIDS) which dominates the existence of life or becomes the obstruction of carrying out a clinical trial (evaluation of functional outcome);
(f) A patient who has a previous disease which confuses neurological scale observation;
(g) A patient of a disease in which a substance which exerts influence upon mind is concerned, a patient with a history of dementia that daily life actions cannot be carried out or a patient with a history of alcohol abuse;
(h) A patient who joined other clinical trial within 30 days before the entry to the trial;
(i) A patient who is pregnant or is in the lactational period. A female patient who was not pregnant at the time of the commencement of this trial (human β-chorionic gonadotropin in urine is negative) and can agree with keeping continence during the administration period can join the trial;
(j) A patient who has disorder in liver function: bilirubin > 2 mg/ml and/or ascites;
(k) A patient who has disorder in renal function : serum creatinine > 2 mg/dl;
(l) A patient of a decompensated congestive heart failure or of a decompensated congestive heart failure which requires periodical administration of a parenteral cardiotonic or who frequently causes a decompensation;
(m) A patient whose PR interval on electrocardiogram before the commencement of administration exceeds 220 milliseconds or a patient whose QT interval corrected by the base line exceeds 480 milliseconds, or a patient who has a medical record on ventricular arrhythmia or an atrioventricular block of Mobitz 1 type or more;
(n) A patient who previously joined a clinical trial on (2R)-2-propyloctanoic acid.

### [DOSAGE AND ADMINISTRATION]

A total of 5 times for one hour intravenous continuous administration at intervals of about 24 hours in each group (administration period: 5 days).
(1) (2R)-2-propyloctanoic acid 4 mg/kg/hr;
(2) (2R)-2-propyloctanoic acid 10 mg/kg/hr;
(3) Placebo.

### [NUMBER OF SUBJECTS]

(1) (2R)-2-propyloctanoic acid 4 mg/kg/hr administration group: 269 persons;
(2) (2R)-2-propyloctanoic acid 10 mg/kg/hr administration group: 291 persons;
(3) Placebo administration group: 281 persons.

### [EFFICACY EVALUATION ITEMS]

### (1) NIH Stroke Scale (NIHSS)

Evaluation method: Symptoms of patients were evaluated in accordance with the aforementioned list of marks of NIH Stroke Scale, about 1 month after or about 3 months after the commencement of administration.

### (2) Modified Rankin Scale (mRS)

Evaluation method: Symptoms of patients were scored by classifying the symptoms of patients into the following grades of from 0 to 5, about 1 month after or about 3 months after the commencement of administration.
0 : No symptoms at all.
1 : No significant disability despite symptoms; able to carry out all usual duties and activities.
2 : Slight disability; unable to carry out all previous activities, but able to look after own affairs without assistance.
3 : Moderate disability; requiring some help, but able to walk without assistance.
4 : Moderately severe disability; unable to walk without assistance and unable to attend to own bodily needs without assistance.
5 : Severe disability; bedridden, incontinent and requiring constant nursing care and attention.

### (3) Barthel Index (EI)

Evaluation method: Symptoms of patients were scored in accordance with the following list of marks, about 1 month after or about 3 months after the commencement of administration.
1. Feeding
   10 : The patient is independent, can equip with self-helping means and the like. The patient can finish eating within a standard time.
   5 : Partial help (*e.g.*, have an accompanying dish cut into pieces).
   0 : Total help.
2. Transfer from wheelchair to bed
   15 : Independent, including operation of the brake and footrest of wheelchair (includes independent walking).
   10 : Requires slight partial help or watch.
   5 : Sitting is possible but with almost total help.
   0 : Total help or impossible.
3. Grooming
   5 : Independent (face washing, hair dressing, tooth brushing, shaving).
   0 : Partial help or total help.
4. Toilet Use
   10 : Independent, including handling of clothes and clear up. When a bedside commode or the like is used, its washing is also included.
   5 : Partial help. Requires support of the body and for clothes and clear up.
   0 : Total help or impossible.
5. Bathing
   5 : Independent.
   0 : Partial help or total help.
6. Mobility
   15 : Walks 45 m or more. Does not matter whether or not a supporting tool is used (excluding a wheelchair and a walker).
   10 : Helped walking of 45 m or more. Includes the use of a walker.
   5 : Handling of 45 m or more with a wheelchair is possible in the case of walking impossible.
   0 : Other than the above.
7. Stairs
   10 : Independent (a handrail or walking stick can be used).
   5 : Requires help or watch.
   0 : Impossible.
8. Dressing
   10 : Independent. Put on and off of shoes, zippers and equipment are included.
   5 : Partial help. Within a standard period of time. More than half of it can be carried out in person.
   0 : Other than the above.
9. Bowels
   10 : No incontinence. Can handle enema and suppository.
   5 : Occasional incontinence. A person who requires help for handling enema or suppository is also included.
   0 : Other than the above.
10. Bladder
   10 : No incontinence. Can handle a bedpan.
   5 : Occasional incontinence. A person who requires help for handling a bedpan is also included.
   0 : Other than the above.

### (4) Glasgow Outcome Scale (GOS)

Evaluation method: Symptoms of patients were scored by classifying into the following grades of from 1 to 5, about 1 month after or about 3 months after the commencement of administration.
1 : Good Recovery: general personal life can be carried out disregard of the presence or absence of minimum neurological disorder.
2 : Moderate Disability: Has a neurological or intellectual and personality disorder, but can make a living without assistance.
3 : Severe Disability: remains conscious, but entirely requires assistance for making daily life.
4 : Vegetative state.
5 : Death.

### [SAFETY EVALUATION ITEMS]

Frequency of adverse event and contents thereof (symptom, causal relation and the like)

### [ANALYSIS]

Based on the results of the above-mentioned efficacy evaluation items and safety evaluation items, therapeutic effect of the intravenous continuous administration of (2R)-2-propyloctanoic acid to cerebral infarction patients was evaluated. Additionally, in order to examine the therapeutic effect more in detail, improving effect of the intravenous continuous administration of (2R)-2-propyloctanoic acid for each of (a) [Arm strength], (b) [Grip], (c) strength of the legs [Leg], (d) strength of the ahead of the ankle [Ankle/foot], and (e) language [Naming] was evaluated after about 3 months from the commencement of administration, using the items 1a, 1b, 1c, 1d and 4a of the Stroke Impact Scale (SIS). In this connection, the Stroke Impact Scale is a conventionally known evaluation method and can be obtained from an internet <URL: www.chrp.org/, www.strokecenter.org/, www2.kumc.edu/coal/> and the like. Specifically, the evaluation of 1a, 1b, 1c and 1d is carried out by a mode of selecting any one of the answers, A lot of strength, Quite a bit of strength, Some strength, A little strength and No strength at all, for the following questions
1. In the past week, how would you rate the strength of your ...
   a. Arm that was most affected by your stroke?
   b. Grip of your hand that was most affected by your stroke?
   c. Leg that was most affected by your stroke?
   d. Foot/ankle that was most affected by your stroke?
and five scores, four scores, 3 scores, 2 scores and 1 score are given in response to the respective answers.

Additionally, the evaluation of 4a is carried out by a mode of selecting any one of the answers, Not difficult at all, A little difficult, Somewhat difficult and Very difficult and Extremely difficult, for the following questions
4. In the past week, how difficult was it to ...
   a. Say the name of someone who was in front of you?
and five scores, four scores, 3 scores, 2 scores and 1 score are given in response to the respective answers.

### [RESULTS]

The results are shown in the following.

### [EFFICACY EVALUATION ITEMS]

When ratio of the number of patients whose scores by the modified Rankin Scale (mRS) after 3 months from the commencement of administration became 0 to 3 was compared between the placebo administration group and the (2R)-2-propyloctanoic acid 10 mg/kg/hr administration group, it was 23.6% in the placebo administration group and 31.5% in the (2R)-2-propyloctanoic acid 10 mg/kg/hr administration group, in the class of patients to which the administration was carried out after a lapse of time of from 5.5 hours to 6 hours from the onset of the disease. Additionally, it was 28.8% in the placebo administration group and 39.0% in the (2R)-2-propyloctanoic acid 10 mg/kg/hr administration group, in the class of patients of less than 80 years to which the administration was carried out after a lapse of time of from 5.5 hours to 6 hours from the onset of the disease, and it was 34.5% in the placebo administration group and 46.0% in the (2R)-2-propyloctanoic acid 10 mg/kg/hr administration group, in the class of patients of less than 80 years who showed an NIH Stroke Scale score of from 11 to 18 to which the administration was carried out after a lapse of time of from 5.5 hours to 6 hours from the onset of the disease.

Since high reactivity was found in the (2R)-2-propyloctanoic acid administration group in comparison with the placebo group, a result was obtained in that (2R)-2-propyloctanoic acid is effective for the treatment of acute cerebral infarction.

The following Table 1 shows the effect of (2R)-2-propyloctanoic acid intravenous continued administration to improve (a) [Arm strength], (b) [Grip], (c) strength of the legs [Leg], (d) strength of the ahead of the ankle [Ankle/foot], and (e) language [Naming], respectively, by using the Stroke Impact Scale. In this case, the group P represents a placebo administration group, and the group L represents a (2R)-2-propyloctanoic acid 4 mg/kg/hr administration group, and the group H represents a (2R)-2-propyloctanoic acid 10 mg/kg/hr administration group. The evaluation result of each item was shown by a form of "(the number of patients having an evaluation score of 4 or more)/(the number of patients evaluated)". In this connection, since the class of patients who showed a score of 32 or less on the NIH Stroke Scale after a lapse of time of 5 hours or more from the onset of the disease were analyzed as the object of the analysis, the number of resulting patients is used as the number of patients to be evaluated. The percentage (%) in the parentheses shows ratio of the number of patients to be tested who showed an evaluation score of 4 or more among the number of evaluated patients to be evaluated.

**Table 1**

| | Arm strength | Grip | Leg | Ankle/foot | Naming |
|---|---|---|---|---|---|
| Group P | 25/93 | 28/93 | 43/93 | 39/93 | 54/93 |
| | (27%) | (30%) | (46%) | (42%) | (58%) |
| Group L | 34/88 | 36/88 | 46/88 | 39/88 | 58/88 |
| | (39%) | (41%) | (52%) | (44%) | (66%) |
| Group H | 44/100 | 46/100 | 59/100 | 60/100 | 65/100 |
| | (44%) | (46%) | (59%) | (60%) | (65%) |

As is clear also from the above-mentioned Table 1, in each items of (a) [Arm strength], (b) [Grip], (c) strength of the legs [Leg], (d) strength of the ahead of the ankle [Ankle/foot], and (e) language [Naming], ratio of the number of patients to be tested having an evaluation score of 4 or more was high in the (2R)-2-propyloctanoic acid administration group in comparison with the placebo administration group. Thus, excellent improving effect was found for each of these items by the administration of 4 mg/kg/hr or 10 mg/kg/hr of (2R)-2-propyloctanoic acid. Accordingly, it can be said that (2R)-2-propyloctanoic acid is effective for the improvement of symptoms of acute cerebral infarction.

### [SAFETY EVALUATION ITEMS]

A severe adverse events having a different frequency between the (2R)-2-propyloctanoic acid administration group and placebo administration group was not found. Additionally, a side effect in which its frequency increases depending on the dose of (2R)-2-propyloctanoic acid was not found, too.

### Example 2:

As a clinical trial, a double blind placebo-controlled parallel group study was carried out under the following conditions using patients of acute cerebral infarction as the object.

### [STUDY POPULATION]

A total of 448 patients of acute cerebral infarction who meets the following inclusion criteria and does not meet any of the exclusion criteria.

### <Inclusion Criteria>

(a) Sex: Ignored;
(b) Age: 20 or over but under 80 (at the time of obtaining consent);
(c) Body weight: Less than 100 kg;
(d) Inpatient or outpatient: Inpatient (at the time of the commencement of the test);
(e) A patient whose period of time from the onset of cerebral infarction until commencement of administration is 72 hour or less;
(f) A patient who was diagnosed as cerebral infarction by the clinical symptoms or by a computed tomography (CT or MRI) just after admission to a hospital and has an evident motor paralysis based on a cerebrovascular damage;
(g) A patient who did not require help before the onset of cerebral infarction of this time;
(h) A patient whose modified Rankin Scale became 3 or more by the onset of cerebral infarction of this time;
(i) A patient whose level of consciousness is from 7 to 14 by the Glasgow Coma Scale and is from I-1 to II-30 by the Japan Coma Scale;
(j) A patient whose Japan Coma Scale before the administration is 15 or less and whose NIH Stroke Scale before the administration is 22 or less;

### <Exclusion Criteria>

(a) A patient who is under a deep coma state and considered to be difficult to save life;
(b) A patient unsuited for evaluation of drug potency due to severe agitation of the symptom;
(c) A patient who is scheduled to undergo an operation (intravascular operation and revascularization are included) which is presumed to exert influence upon prognosis of the patient;
(d) A patient who used a concomitant use-banned drug (a thrombolytic agent, cerebral metabolic agent, cerebral vasodilator drug, anticoagulant, anti-platelet drug, brain protecting agent or the like) during a period of from the onset of cerebral infarction to the commencement of the test;
(e) A patient having a total bilirubin of 3.0 mg or more and a patient whose AST (GOT) or ALT (GPT) is 2.5 times or more of the normal value (or 100 IU/L or more);
(f) A patient having a serum creatinine value of 2 mg/dL;
(g) A patient of severe heart failure (NYHA classification class III (a heart disease patient having high degree limitation for physical activity; fatigue, palpitation, difficulty of breathing and angina pectoris occur even by a light physical activity in daily life) or more);
(h) A patient having a past history of severe allergy by a drug and the like;
(i) A patient who is pregnant or a patient having a possibility of pregnant or a patient who is in the lactational period;
(j) A patient who did not pass 6 months after completion of the administration of all other investigational drugs;
(k) Other patients who are judged unsuitable as the object of investigation by the principal investigator or subinvestigator.

### [DOSAGE AND ADMINISTRATION]

A total of 7 times for one hour intravenous continuous administration at intervals of about 24 hours in each group (administration period: 7 days).
(1) (2R)-2-propyloctanoic acid 0.4 mg/kg/hr;
(2) (2R)-2-propyloctanoic acid 4 mg/kg/hr;
(3) (2R)-2-propyloctanoic acid 8 mg/kg/hr;
(4) placebo.

### [NUMBER OF SUBJECTS]

(1) (2R)-2-propyloctanoic acid 0.4 mg/kg/hr administration group: 102 persons;
(2) (2R)-2-propyloctanoic acid 4 mg/kg/hr administration group: 118 persons;
(3) (2R)-2-propyloctanoic acid 8 mg/kg/hr administration group: 113 persons;
(4) placebo administration group: 115 persons.

### [EFFICACY EVALUATION ITEMS]

Symptoms of patients were evaluated after about 1 month or after about 3 months from the commencement of the administration, in accordance with the methods described in the aforementioned [EFFICACY EVALUATION ITEMS] of Example 1.

### [SAFETY EVALUATION ITEMS]

Frequency of adverse event and contents thereof (symptom, causal relation and the like)

### [ANALYSIS]

Based on the results of the above-mentioned efficacy evaluation items and safety evaluation items, therapeutic effect of the intravenous continuous administration of (2R)-2-propyloctanoic acid to cerebral infarction patients was evaluated.

### [RESULTS]

The results are shown in the following.

### [EFFICACY EVALUATION ITEMS]

When the placebo administration group and the (2R)-2-propyloctanoic acid 8 mg/kg/hr administration group were compared by the modified Rankin Scale (mRS) after 3 months from the commencement of administration, the ratio of mRS occupying 0 to 2 was 45.8% in the placebo administration group and 58.1 % in the (2R)-2-propyloctanoic acid 8 mg/kg/hr administration group. Also, when the placebo administration group and the (2R)-2-propyloctanoic acid 8 mg/kg/hr administration group were compared by the modified Rankin Scale (mRS) after 1 month from the commencement of administration, the ratio of mRS occupying 0 to 2 was 27.8% in the placebo administration group and 45.7% in the (2R)-2-propyloctanoic acid 8 mg/kg/hr administration group. Additionally, when the placebo administration group and the (2R)-2-propyloctanoic acid 8 mg/kg/hr administration group were compared, using the patient group having a Japan Coma Scale (JCS) value of from I-1 to I-3 before the administration, by the modified Rankin Scale (mRS) after 3 months from the commencement of administration, the ratio of mRS occupying 0 to 2 was 50.7% in the placebo administration group and 69.1% in the (2R)-2-propyloctanoic acid 8 mg/kg/hr administration group. Furthermore, when the placebo administration group and the (2R)-2-propyloctanoic acid 0.4, 4 or 8 mg/kg/hr administration group were compared, using the patient group having a Japan Coma Scale (JCS) value of from I-1 to I-3 before the administration and an NIH Stroke Scale value of 7 or more, by the modified Rankin Scale (mRS) after 3 months from the commencement of administration, the ratio of mRS occupying 0 to 2 was 42.4% in the placebo administration group, 48.1 % in the (2R)-2-propyloctanoic acid 0.4 mg/kg/hr administration group, 47.9% in the (2R)-2-propyloctanoic acid 4 mg/kg/hr administration group and 63.0% in the (2R)-2-propyloctanoic acid 8 mg/kg/hr administration group.

Since high reactivity was found in the (2R)-2-propyloctanoic acid administration group in comparison with the placebo administration group for any class of patients as the object, a result was obtained that (2R)-2-propyloctanoic acid is effective for the treatment of acute cerebral infarction.

### [SAFETY EVALUATION ITEMS]

A severe adverse event having a different frequency between the (2R)-2-propyloctanoic acid administration group and the placebo administration group was not found. Additionally, a side effect in which its frequency increases depending on the dose of (2R)-2-propyloctanoic acid was not found, too.

### Example 3:

As a clinical trial, a double blind placebo-controlled parallel group study was carried out under the following conditions using patients of acute cerebral infarction as the obj ect.

### [STUDY POPULATION]

From about 720 to 750 patients of acute cerebral infarction who meets the following inclusion criteria and does not meet any of the exclusion criteria.

### <Inclusion Criteria>

(a) Sex: Ignored;
(b) Age: 20 or over but under 80 (at the time of obtaining consent);
(c) Body weight: Less than 100 kg;
(d) Inpatient or outpatient: Inpatient (at the time of the commencement of the test);
(e) A patient whose period of time from the onset of cerebral infarction until commencement of administration is 72 hour or less;
(f) A patient who was diagnosed as cerebral infarction by the clinical symptoms or by a computed tomography (CT or MRI) just after admission to a hospital and has an evident motor paralysis based on a cerebrovascular damage;
(g) A patient who became necessary to have help by the onset of cerebral infarction of this time;
(h) A patient whose level of consciousness just before the administration is from 0 to 1-3 by the Japan Coma Scale;
(i) A patient whose NIH Stroke Scale before the administration is from 7 to 22 (7 or more and 22 or less).

### <Exclusion Criteria>

(a) A patient unsuited for evaluation of drug potency due to severe agitation of the symptom;
(b) A patient who had a disorder which corresponds to a value of 3 or more by the modified Rankin Scale before the onset of cerebral infarction of this time;
(c) A patient who is scheduled to undergo an operation (intravascular operation and revascularization are included) which is presumed to exert influence upon prognosis of the patient;
(d) A patient who used a concomitant use-banned drug (a thrombolytic agent, anticoagulant, anti-platelet drug, brain protecting agent, cerebral metabolism improving agent, cerebral circulation improving agent or the like) during a period of from the onset of cerebral infarction to the commencement of the test;
(e) A patient having a total bilirubin of 3.0 mg or more or a patient whose AST (GOT) or ALT (GPT) is 2.5 times or more of the normal value (or 100 IU/L or more);
(f) A patient having a serum creatinine value of 2.0 mg/dL;
(g) A patient of severe heart failure (NYHA classification class III (a heart disease patient having high degree limitation for physical activity; fatigue, palpitation, difficulty of breathing and angina pectoris occur even by a light physical activity in daily life) or more);
(h) A patient having a past history of severe allergy by a drug and the like;
(i) A patient who is pregnant or is in the lactational period, or a patient having a possibility of pregnant, or a patient who wish to become pregnant during the investigation period;
(j) A patient who did not pass 4 months after completion of the administration of all other investigational drugs;
(k) A patient who was administered with (2R)-2-propyloctanoic acid in the past;
(l) Other patients who are judged unsuitable as the object of investigation by the principal investigator or subinvestigator.

### [DOSAGE AND ADMINISTRATION]

A total of 7 times for one hour intravenous continuous administration at intervals of about 24 hours in each group (administration period: 7 days).
(1) (2R)-2-propyloctanoic acid 8 mg/kg/hr;
(2) (2R)-2-propyloctanoic acid 12 mg/kg/hr;
(3) placebo.

### [NUMBER OF SUBJECT]

(1) (2R)-2-propyloctanoic acid 8 mg/kg/hr administration group: about 240 to 250 persons;
(2) (2R)-2-propyloctanoic acid 12 mg/kg/hr administration group: about 240 to 250 persons;
(3) placebo administration group: about 240 to 250 persons.

### [EFFICACY EVALUATION ITEMS]

Symptoms of patients were evaluated after about 1 month or after about 3 months from the commencement of the administration, in accordance with the methods described in the aforementioned [EFFICACY EVALUATION ITEMS] of Example 1.

### [SAFETY EVALUATION ITEMS]

Frequency of adverse event and contents thereof (symptom, causal relation and the like)

### [ANALYSIS]

Based on the results of the above-mentioned efficacy evaluation items and safety evaluation items, therapeutic effect of the intravenous continuous administration of (2R)-2-propyloctanoic acid to cerebral infarction patients was evaluated.

### [RESULTS]

The results are shown in the following.

### [EFFICACY EVALUATION ITEMS]

By administering 8 mg/kg/hr or 12 mg/kg/hr of (2R)-2-propyloctanoic acid, excellent improving effects of the items shown in the efficacy evaluation items can be found.

### [SAFETY EVALUATION ITEMS]

A severe adverse event having a different frequency between the (2R)-2-propyloctanoic acid administration group and the placebo administration group is not found. Additionally, a side effect in which its frequency increases depending on the dose of (2R)-2-propyloctanoic acid is not found, too.

### Formulation Examples:

Production of (2R)-2-propyloctanoic acid-containing injections

### Formulation Example 1

| | |
|---|---|
| (2R)-2-propyloctanoic acid | 2.0 kg |
| Trisodium phosphate•12H₂O | 3.54 kg |

Each of the above components was added to water for injection, and the volume was adjusted to 40 liters using distilled water for injection. It was made into a homogeneous solution, filtered through a sterile filter (Durapore 0.22 µm membrane), dispensed in 2 ml portions into plastic ampoules and then subjected to autoclave sterilization (123 °C, 15 minutes) to obtain 20,000 ampoules containing 100 mg of the active ingredients per one ampoule.

### Formulation Example 2

| | |
|---|---|
| (2R)-2-propyloctanoic acid | 5.0 kg |
| Disodium hydrogenphosphate•12H₂O | 8.0 kg |
| Sodium hydroxide | 1.03 kg |

The above-mentioned respective components were added to distilled water for injection and pH was adjusted to 8.4 to 9.0 by adding suitable amount of sodium hydroxide. The total volume was adjusted to 100 L using distilled water for injection. After making homogeneous solution, it was filtered through a sterile filter (0.22 µm membrane, manufactured by Durapore) and dispensed in 1 ml portions into polypropylene plastic ampoules (brow fill sealing). By subjecting the resulting ampoules to autoclave sterilization (123 °C, 5 minutes), 95,000 ampoules containing 50 mg of the active ingredient per one ampoule were obtained.

### [INDUSTRIAL APPLICABILITY]

The "method for treating acute cerebral infarction, which comprises administration of an effective amount of (2R)-2-propyloctanoic acid or a salt thereof to a mammal after a lapse of time of from about 5 hours to about 72 hours from the onset of the disease" and the "agent for treating acute cerebral infarction comprising (2R)-2-propyloctanoic acid or a salt thereof which is used for administration after a lapse of time of from about 5 hours to about 72 hours from the onset of the disease" to be used in the method, which are disclosed in the invention, are safe and can markedly improve various symptoms accompanied by cerebral infarction. The agent for treating acute cerebral infarction comprising (2R)-2-propyloctanoic acid or a salt thereof which is used for administration after a lapse of time of from about 5 hours to about 72 hours from the onset of the disease is really useful as a pharmaceutical, since when administered in accordance with the use and dose disclosed in the present invention, it shows its effect even in a case after a lapse of time of 3 hours or more from the onset of the disease which was difficult to be treated by the conventional therapeutic agents.

## Claims

1. An agent for treating acute cerebral infarction, comprising (2R)-2-propyloctanolc acid or a salt thereof, which is used for administration after a lapse of time of from about 5 hours to about 72 hours from the onset of the disease.

2. The agent according to claim 1, wherein the treatment of acute cerebral infarction is improvement of neurological symptom, impairment of activities of daily living and/or functional disorder, accompanying acute cerebral infarction.

3. The agent according to claim 1, wherein the treatment of acute cerebral infarction is improvement of disturbed consciousness, lalopathy and/or impaired limb motor function, accompanying acute cerebral infarction.

4. The agent according to claim 1, wherein the acute cerebral infarction is mild or moderate.

5. The agent according to claim 4, wherein the acute cerebral infarction is moderate.

6. The agent according to claim 1, wherein the acute cerebral infarction is defined as a score of 22 or less on NIH Stroke Scale.

7. The agent according to claim 6, wherein the acute cerebral infarction is defined as a score of 7 to 22 on NIH Stroke Scale.

8. The agent according to claim 7, wherein the acute cerebral infarction is defined as a score of 7 to 18 on NIH Stroke Scale.

9. The agent according to claim 1, which is used for intravenous administration.

10. The agent according to claim 9, wherein the amount per dose of (2R)-2-propyloctanoic acid or a salt thereof for intravenous administration is within a range of from about 100 mg to about 2000 mg in terms of (2R)-2-propyloctanoic acid.

11. The agent according to claim 10, which is used for continuous administration.

12. The agent according to claim 11, which is used for infusion bag administration.

13. The agent according to claim 11, wherein the continuous administration is carried out for 30 minutes to 90 minutes.

14. The agent according to claim 9, wherein the dose for intravenous administration per I kg of body weight of a patient is within a range of from about 2 mg to about 20 mg.

15. The agent according to claim 14, wherein the dose for intravenous administration per 1 kg of body weight of a patient is about 4 mg, about 6 mg, about 8 mg, about 10 mg, about 12 mg, about 15 mg or about 18 mg.

16. The agent according to claim 15, wherein the dose for intravenous administration per 1 kg of body weight of a patient is about 8 mg or about 10 mg.

17. The agent according to claim 1, wherein the administration period is from 1 day to 100 days from the administration after a lapse of time of from about 5 hours to about 72 hours from the onset of the disease.

18. The agent according to claim 17, wherein the administration period is from 1 day to 10 days from the administration after a lapse of time of from about 5 hours to about 72 hours from the onset of the disease.

19. The agent according to claim 18, wherein the administration period is from 5 days to 7 days from the administration after a lapse of time of from about 5 hours to about 72 hours from the onset of the disease.

20. The agent according to claim 1, wherein the administration is carried out once a day.

21. An agent for treating an acute cerebral infarction defined as a score of 22 or less on NIH Stroke Scale, comprising (2R)-2-propyloctanoic acid,
which is used for continuous administration for 30 minutes to 90 minutes once a day using infusion bag in an amount per dose of from about 2 mg to about 20 mg per 1 kg of body weight of a patient for intravenous administration,
in which the administration period is from 1 day to 10 days from the administration after a lapse of time of from about 5 hours to about 72 hours from the onset of the disease.

22. An agent for improving neurological symptom, impairment of activities of daily living and/or functional disorder, accompanying acute cerebral infarction defined as a score of 7 to 22 on NIH Stroke Scale, comprising (2R)-2-propyloctanoic acid,
which is used for continuous administration for 30 minutes to 90 minutes once a day using infusion bag in an amount per dose of from about 8 mg or about 10 mg per 1 kg of body weight of a patient for intravenous administration,
in which the administration period is from 5 day to 7 days from the administration after a lapse of time of from about 5 hours to about 72 hours from the onset of the disease.

23. An agent for improving disturbed consciousness, lalopathy and/or impaired limb motor function, accompanying acute cerebral infarction defined as a score of 11 to 18 on NIH Stroke Scale, comprising (2R)-2-propyloctanoic acid,
which is used for continuous administration for 30 minutes to 90 minutes once a day using infusion bag in an amount per dose of about 8 mg or about 10 mg per 1 kg of body weight of a patient for intravenous administration,
in which the administration period is from 5 day to 7 days from the administration after a lapse of time of from about 5 hours to about 72 hours from the onset of the disease.

24. A method for treating acute cerebral infarction, which comprises administration of an effective amount of (2R)-2-propyloctanoic acid or a salt thereof to a mammal after a lapse of time of from about 5 hours to about 72 hours from the onset of the disease.

25. Use of (2R)-2-propyloctanoic acid or a salt thereof for the manufacture of an agent for treating acute cerebral infarction which is used for administration after a lapse of time of from about 5 hours to about 72 hours from the onset of the disease.
